# DEMANDE DE BREVET EUROPEEN

(11) **EP 1 245 220 A1**
(43) Date de publication de la demande: **02.10.2002**
(21) Numéro de dépôt: 02356056.8
(22) Date de dépôt: 26.03.2002
(51) Int. Cl.: A61K 7/155

(54) **Composition de cire a epiler**

(30) Priorité: 30.03.2001 FR 0104407
(71) Demandeur: SEB S.A., 69130 Ecully (FR)
(72) Inventeur: Paget, Monique, 38460 Cremieu (FR); Khenniche-Nedjadi, Samira, 69100 Villeurbanne (FR)
(74) Mandataire: Kiehl, Hubert

(57) **Abrégé**

Composition de cire à épiler réalisée à base d'une cire du type colophane. Selon l'invention, ladite base de cire du type colophane est mélangée avec une solution aqueuse et au moins un tensioactif.

## Description

La présente invention est relative au domaine technique des cires dites basses températures utilisées pour l'épilation. Cette catégorie de cires peut être utilisée dans des distributeurs de cire tels que par exemple des applicateurs de cire à rouleau.

Des distributeurs de cire à épiler du type applicateur à rouleau sont connus notamment des documents FR 2 520 601 et EP 0 629 366. Chacun de ces appareils comporte un réservoir contenant la cire à épiler associé à des moyens de chauffe et un rouleau prévu pour distribuer la cire ramollie par la température en couche mince sur la peau. Les distributeurs de cire permettent de garantir une totale sécurité en matière de température d'application de la cire sur la peau. Les rouleaux applicateurs apportent un confort évident d'utilisation par un geste simple, pratique et très précis permettant la dépose de fines bandes de cire sur la peau.

On connaît dans l'état de la technique deux principales familles de cires à épiler utilisées avec de tels distributeurs.

Ainsi, il existe une première famille de cires à épiler dont la composition à base de sucre leur confère la solubilité dans l'eau. Les formules des cires au sucre, caramélisées ou non, telles que décrites dans les documents EP-649646, FR-2541894, FR-2626468, FR-2692144, ES-2080701, CH-604711, CH-670044, US-2417882, US-4832949, US-4842610, US-5158765, GB-1242083, GB-2231494, CA-895834, CA-1185186, CA-2149347 sont essentiellement composées de polysaccharides ou monosaccharides (saccharose, glucose, fructose, dextrose, maltose), d'eau et de quelques pourcentages d'acide citrique.

Les cires à base de sucre agissent comme des colles dans lesquelles les poils sont englués mais non emprisonnés car la matière ne durcit pas au refroidissement. Ces cires nécessitent l'usage de bandes supports telles que des bandes de tissu, de papier ou de cellophane qui sont appliquées sur les couches de cire déposées sur la peau, et arrachées avec les poils qui ont adhérés à la bande support. Les cires au sucre sont chauffées à une température prédéterminée afin d'améliorer leur viscosité et donc leur adhésion aux poils. Malgré leur mise en température assez rapide, ces cires ne sont pas très efficaces quant à l'enrobage et l'arrachage des poils.

Par ailleurs, on connaît des documents DE 1 036 472, FR 2 105 039 ou FR2 798 064 des cires au sucre comportant une faible proportion d'additif, par exemple de la colophane, additif qui est utilisé en tant qu'agent plastifiant ou adhésif. Si, certes, cet additif est censé améliorer l'adhérence aux poils d'une cire au sucre classique, il ne modifie pas le comportement de cette cire qui est celui d'une colle appliquée avec ou sans chauffage préalable sur la peau et retirée ensuite en utilisant une bande support. Ces cires sont également d'une efficacité limitée.

On connaît également dans l'état de la technique une autre famille de cires dont la composition est à base de résine du type colophane, cires qui sont très efficaces pour l'épilation des poils sur l'ensemble du corps. Ces cires dites basse température, telles que celles connues du document EP 0 299 816 présentent l'avantage d'être autoporteuses. De telles cires sont d'abord chauffées afin de passer d'une consistance solide à une consistance pâteuse ou fluide et elles sont ensuite déposées en couche mince sur la peau à l'aide d'un distributeur à rouleau. La couche de cire une fois solidifiée emprisonne les poils et peut être retirée sans utiliser de bandes supports. Tout en étant efficaces et d'une utilisation facile, ces cires présentent, toutefois, une très mauvaise conductibilité thermique elles sont donc très longues à chauffer, ce qui rend l'épilation laborieuse.

Le but de la présente invention est de remédier au moins en partie aux inconvénients précités et de proposer une composition épilatoire du type colophane nécessitant un moindre temps de chauffage, tout en étant très efficace et facile à utiliser.

Un autre but de l'invention est une cire à épiler sous forme d'émulsion stable dans le temps et en température, sans qu'elle soit agressive pour la peau, tout en ayant de bonnes propriétés de dépose sur la peau, d'adhérence aux poils et qui puisse être ensuite enlevée facilement, avec les poils à épiler, sans utiliser de bande support.

Un but supplémentaire de l'invention est une composition de cire à épiler présentant une efficacité optimisée pour un moindre coût de fabrication.

Ces buts sont atteints avec une composition de cire à épiler réalisée à base d'une cire du type colophane, du fait que ladite base de cire du type colophane est mélangée avec une solution aqueuse et au moins un tensioactif.

Par composition de cire à épiler réalisée à base d'une cire du type colophane on désigne un mélange ayant comme principal ingrédient une cire du type colophane ou comportant majoritairement une cire du type colophane.

Par cire du type colophane on comprend une composition épilatoire du type résinique, c'est à dire à base de colophane (comprenant des acides résiniques) ou de colophane modifiée, de cire d'abeille ou de paraffine et comportant, éventuellement, un élastomère ou d'autres thermoplastiques. Les dérivées de colophane peuvent être, par exemple, l'Hydrogral™, le Polygral™, la Granolite™ de la société DRT.

Une telle cire se trouve à l'état solide à la température ambiante et elle doit être chauffée à environ 70 -80° C pour la rendre fluide en vue de son application sur la peau. De par sa mauvaise conductibilité thermique, le temps de mise à disposition d'une telle composition épilatoire s'avère être très long. Or, on a constaté de manière surprenante que l'addition, dans certaines proportions, d'une solution aqueuse à une composition de cire du type colophane favorise la conductivité thermique ce qui permet de réduire de manière significative le temps de chauffage du mélange obtenu.

Un mélange d'un composant liposoluble avec un composant hydrosoluble présente une faible stabilité dans le temps et pour cette raison il s'avère utile de rajouter un ou plusieurs tensioactifs. Les tensioactifs sont des substances amphiphiles possédant une affinité pour les phases lipophiles et en même temps une affinité pour les phases hydrophiles d'une émulsion, ce qui leur permet de rendre compatibles les deux phases en se disposant autour des globules dispersés d'une émulsion. Ainsi, l'addition d'un ou plusieurs tensioactifs, notamment du type eau dans huile, dans les proportions adéquates autorise de manière avantageuse la réalisation de mélanges stables.

Avantageusement, la composition de la cire à épiler de l'invention comprend 60 à 85 % en poids de cire du type colophane, 25 à 40 % en poids de solution aqueuse et 0.2 à 3 % en poids de tensioactif ou d'un mélange de tensioactifs.

Une telle composition permet de mélanger une base de cire du type colophane, qui est lipophile, avec une solution aqueuse en présence d'un ou plusieurs tensioactifs et d'obtenir une émulsion stable, apte à conserver ses propriétés dans le temps. Cette composition épilatoire nécessite un temps de mise en température réduit par rapport à une cire du type colophane classique, tout en étant très efficace pour l'épilation. Une telle composition garde toutefois son comportement de cire du type colophane, elle reste donc solide ou pâteuse à la température ambiante et elle nécessite un chauffage en vue de son application sur la peau, et peut ensuite être retirée de la peau par arrachage, sans utilisation de bande support.

Dans un premier mode de réalisation de l'invention, ladite solution aqueuse est une cire à épiler hydrosoluble comportant au moins un sucre.

Selon ce mode préféré de réalisation de l'invention, la composition épilatoire est constituée principalement par un mélange, dans des proportions adéquates, de deux cires à épiler, une cire du type colophane et une cire à base de sucre. Lors de nombreux tests effectués, il a été constaté de manière surprenante que l'épilation avec une composition épilatoire obtenue par ce mélange de deux cires est bien plus efficace que celle effectuée avec chaque type de cire du mélange .

Cette composition présente également plus de facilité à la dépose sur la peau, un arrachage plus aisé, sans utiliser de bande support, tout en laissant la peau nette, sans résidus, ni poils superflus.

Utilement, ledit sucre est choisi dans le groupe comprenant le saccharose, le fructose, le glucose, le miel, le sirop d'érable, des sucres invertis, seuls ou en mélange.

De manière générale, le sucre de base est composé majoritairement de saccharose. On peut lui additionner du miel, des monosaccharides ou des polysaccharides. Afin de pouvoir utiliser le saccharose dans une composition épilatoire, il doit être décomposé totalement ou en partie par hydrolyse en ses deux composants: le glucose et le fructose. Le mélange de ces deux monosaccharides est appelé sucre inverti. L'hydrolyse du saccharose ou inversion du saccharose est catalysée, en présence d'eau, par un acide faible (par exemple l'acide citrique), ou un acide dilué dans l'eau ( par exemple l'acide chlorhydrique) ou encore un produit naturel comme le jus de citron. L'hydrolyse du saccharose évite la recristallisation du sucre, très néfaste pour un produit utilisé dans l'épilation.

Dans un deuxième mode de réalisation de l'invention, ladite solution aqueuse comprend un mélange d'eau et d'amidon ou de pulpe de cellulose.

Cette solution aqueuse, mélangée à une composition épilatoire du type colophane, permet de réduire le temps de passage de l'état solide à l'état pâteux lors du chauffage du mélange, tout en améliorant sa viscosité pour obtenir une bonne facilité d'étalement sur la peau et assurer un bon arrachage sans utiliser de bandes support.

Avantageusement, la proportion d'eau dans ladite solution aqueuse est comprise entre 10 et 30 % en poids.

Il a été établi, lors des essais effectués qu'une telle proportion d'eau assure un bon compromis entre les propriétés thermiques et la viscosité de la composition épilatoire obtenue.

Utilement, ledit tensioactif possède une valeur HLB comprise entre 4 et 10.

Une émulsion est un mélange de deux liquides thermodynamiquement instable. Les tensioactifs permettent de rendre ses mélanges métastables. Lesdits tensioactifs sont caractérisés par le HLB. Par HLB on comprend la balance hydrophile-lipophile d'un tensioactif, ce qui détermine le rapport ou l'importance respective des chaînes grasse et hydrophile, sa valeur étant d'autant plus élevée que le surfactant est plus hydrophile. Ainsi, les substances ayant un HLB bas, c'est-à-dire inférieur à 6, ont un comportement principalement lipophile et orientent les émulsions dans le sens hydrophile/lipophile. Un HLB supérieur à 8 et inférieur à 18 caractérise principalement les substances utilisées comme émulsionnants lipophile/hydrophile. Par conséquent, un tensioactif dont le HLB compris entre 4 et 10 assure avantageusement la stabilisation des mélanges de l'invention.

Avantageusement, ledit tensioactif est du type non ionique.

Un tensioactif non ionique se caractérise par une liaison autre que ionique unissant leur pôle hydrophile à leur pôle lipidique. Ainsi, le tensioactif peut comporter une liaison ester, résultant de la combinaison d'acide gras sur des alcools, ou une liaison éther qui résulte de la combinaison d'un alcool gras et d'un alcool hydrophile. On préfère cependant les tensioactifs à liaison ester possédant un HLB faible, car ils sont mieux tolérés par la peau que les surfactifs à liaison éther. De tels tensioactifs peuvent être, à titre d'exemple : des esters de glycol, des esters de sorbitanne, des esters de sorbitanne polyoxyéthylénés ou des esters de saccharose.

De préférence, ledit tensioactif est un émulsionnant glucolipidique d'origine végétale.

Un tel tensioactif est obtenu à base de glucides d'origine végétale et de corps gras également d'origine végétale par mélange d'un alcool gras à chaîne longue et un tensioactif dérivé du sucre. Le produit final est composé d'un glucolipidique à caractère hydrophile et d'une chaîne grasse à caractère lipophile. Un tel tensioactif présente de très bonnes propriétés émulsionnantes vis-à-vis d'une plage large de phases grasses, tout en ayant un comportement cosmétique très agréable et une très bonne tolérance pour la peau. De surcroît, un tel tensioactif est totalement exempt de réactifs chimiques toxiques et il est complètement biodégradable.

Dans une variante préférée de réalisation de l'invention, la composition de cire à épiler comprend : 72,5 % en poids de cire du type colophane, 26,5 % en poids de cire à base de sucre et 1 % en poids d'un émulsionnant du type alkylpolyglucoside.

Cette composition assure le meilleur compromis entre le temps de transformation de l'état solide à l'état pâteux et l'efficacité de l'épilation. Le tensioactif est un alkylpolyglucoside obtenu à base de glucides d'origine végétale ayant de bonnes propriétés stabilisantes, de non-toxicité, de douceur vis-à-vis de la peau et il favorise l'hydratation de la peau. Un tel tensioactif peut être, par exemple, le MONTANOV™ fabriqué par la société SEPPIC. Son utilisation permet d'obtenir facilement des émulsions stables, par exemple par simple dispersion à chaud par agitation mécanique.

Avantageusement, la composition de cire à épiler de l'invention comprend en outre de 0 à 10 % en poids d'au moins un additif choisi dans le groupe comprenant les parfums, les huiles essentielles, les opacifiants, les antiseptiques, les analgésiques, les antibiotiques, les colorants, les plastifiants, les sels, seuls ou en mélange.

Ces additifs confèrent à la composition épilatoire des propriétés de souplesse, d'aspect, antiseptiques, analgésiques, d'odeur qui rendent l'opération d'épilation plus agréable.

L'invention sera mieux comprise à la lecture de la description ci-après, ainsi qu'à la lecture des exemples pris à titre nullement limitatif.

La composition épilatoire est basée sur une émulsion comportant majoritairement une cire du type colophane et une solution aqueuse, présente dans de plus faibles proportions que la cire du type colophane, l'émulsion étant stabilisée par un agent émulsifiant du type eau dans huile, c'est-à-dire un agent émulsifiant possédant une valeur HLB suffisamment faible pour fournir des émulsions eau dans huile.

Une telle formulation contient 60 à 85 % en poids de cire du type colophane, 25 à 40 % en poids de solution aqueuse et 0.2 à 3 % en poids de tensioactif.

La cire du type colophane peut être par exemple celle décrite dans le document EP 0 299 816 où la composition épilatoire comprend 40 à 80% en poids de colophane naturelle et/ou colophane modifiée, 5 à 15 % en poids d'un composé macro-moléculaire, élastomère et/ou thermoplastique, et 10 à 20 % en poids d'un assouplissant, par exemple la graisse, l'huile ou la cire. Une telle cire à épiler présente une plage de ramollissement comprise entre 70 et 80°C. Cette cire est autoporteuse, c'est-à-dire elle s'enlève facilement de la peau sans utiliser de bande support.

Dans un premier mode de réalisation de l'invention, la solution aqueuse est une cire à base de sucre. Une telle cire contient généralement du saccharose, du fructose et de l'eau. Un exemple de mélange de cire à base de sucre contient 90 à 95 % en poids de sucre(s), 5 à 10 % en poids d'eau et 1 à 2 % en poids d'acide citrique. La cire au sucre peut également contenir des additifs améliorant son pouvoir collant, tel que décrit dans la demande de brevet FR 2 798 064 au nom de la demanderesse. Une telle cire présente une plage de ramollissement comprise entre 50 et 60°C. Cette cire s'enlève de la peau en lui apposant une bande support en coton ou cellophane et en retirant cette bande ayant adhéré à la cire ensemble avec les poils enrobés par la cire.

Dans une variante avantageuse de l'invention on utilise un tensioactif qui fait partie de la famille des APG ou alkylpolyglucosides. Un exemple d'un tel tensioactif est donné dans le document WO 92/06778. Des résultats significatifs ont été obtenus avec le MONTANOV™ L de la société SEPPIC qui est un émulsionnant glucolipidique d'origine végétale possédant un HLB proche de 10, constitué par un mélange d'un alcool à chaîne longue et un sucre, sa formule étant : C14-C22 alkylalcohol et C12-C20 alkylglucoside. Ce tensioactif peut être utilisé comme émulsionnant unique ou en association avec d'autres émulsionnants tels que par exemple d'autres APG comme les esters de polyglycérol, les esters de saccharose, les esters de sorbitol, etc.

D'autres tensioactifs peuvent également être utilisés en mélange, tels que le MONTANE™ 481 et le SIMULSOL™ 989 de la société SEPPIC. Le MONTANE™ 481 est un émulsionnant eau/huile possédant un HLB de 4,5. Il est constitué d'un mélange de cires et de tensioactifs non ioniques.
Le SIMULSOL™ 989 est un émulsionnant non ionique à caractère lipophile possédant un HLB de 6,4.

Dans une variante préférée de réalisation de l'invention, la composition de cire à épiler comprend 72,5 % de cire du type colophane, 26,5 % de cire à base de sucre et 1 % de MONTANOV™ L.

Dans un deuxième mode de réalisation de l'invention, la solution aqueuse est constituée par une suspension ou empois d'amidon ou de pulpe de cellulose.

Une telle formulation contient 70 à 90 % en poids d'amidon, 8 à 28 % en poids d'eau et 2 % en poids de MONTANOV™ L.

Un autre exemple de solution aqueuse est constitué par la formulation contenant 88 % en poids de cellulose, 10 % en poids d'eau et 2 % en poids de MONTANOV™ L.

La mise en oeuvre des mélanges décrits consiste à mélanger à chaud à une température d'environ 90°C, les quantités requises de la phase grasse et de la phase aqueuse respectivement une cire du type colophane et par exemple une cire à base de sucre. Ensuite, les produits de fusion sont mélangés sous agitation à une vitesse d'environ 150 tours /min durant 2 heures à une température de 90 °C. Puis on rajoute le ou les tensioactif(s) sous agitation. Après cet ajout le mélange est maintenu sous agitation pendant 1,5 heures. On obtient ainsi une cire autoporteuse sous forme d'une émulsion métastable. Le produit final est coulé dans des moules. On laisse refroidir, on obtient ainsi les formes et les dimensions prédéterminées.

La composition de cire à épiler de l'invention présente une viscosité comprise entre 1200 et 3000 Pa.s pour une température de 50 ° C , une fréquence de cisaillement de 1Hz et une contrainte de 1000 Pa .

Lors de l'opération de l'épilation, on commence par remplir avec la composition de cire à épiler, le réservoir muni d'un élément chauffant électrique d'un distributeur de cire à rouleau applicateur, distributeur décrit dans le document EP 0 629 366 de la demanderesse. Ensuite on branche l'appareil à une source d'alimentation et on attend le temps nécessaire pour que la composition de cire à épiler passe de l'état solide à un état pâteux en vue de son application sur la peau. Ainsi, il a été constaté qu'avec la composition à épiler de l'invention, le temps de mise à disposition de la cire est réduit d'environ de moitié par rapport au temps de mise à disposition d'une cire du type colophane classique. Une fois cette cire ramollie, elle est appliquée sur la peau d'où elle est retirée facilement sans utiliser de bande support.

D'autres variantes et modes de réalisation de l'invention peuvent être imaginés sans sortir du cadre de ses revendications. Ainsi, à la place de la cire colophane on peut utiliser tout autre type de cire à comportement lipophile issue de mélange de cire d'abeilles, de paraffine, seules ou en mélange avec un élastomère. On peut également utiliser d'autres solutions aqueuses dans certaines proportions, en choisissant les proportions adéquates pour le tensioactif.

## Revendications

1. Composition de cire à épiler réalisée à base d'une cire du type colophane, **caractérisée en ce que** ladite base de cire du type colophane est mélangée avec une solution aqueuse et au moins un tensioactif.

2. Composition de cire à épiler selon la revendication 1, **caractérisée en ce qu'**elle comprend 60 à 85 % en poids de cire du type colophane, 25 à 40 % en poids de solution aqueuse et 0.2 à 3 % en poids de tensioactif ou d'un mélange de tensioactifs.

3. Composition de cire à épiler selon l'une des revendications 1 ou 2, **caractérisée en ce que** ladite solution aqueuse est une cire à épiler hydrosoluble comportant au moins un sucre.

4. Composition de cire à épiler selon la revendication 3, **caractérisée en ce que** ledit sucre est choisi dans le groupe comprenant le saccharose, le fructose, le glucose, le miel, le sirop d'érable, des sucres invertis, seuls ou en mélange.

5. Composition de cire à épiler selon la revendication 1 ou 2, **caractérisée en ce que** ladite solution aqueuse comprend un mélange d'eau et d'amidon ou de pulpe de cellulose.

6. Composition de cire à épiler selon l'une des revendications 3 à 5, **caractérisée en ce que** la proportion d'eau dans ladite solution aqueuse est comprise entre 10 et 30% en poids.

7. Composition de cire à épiler selon l'une des revendications précédentes, **caractérisée en ce que** ledit tensioactif possède une valeur HLB comprise entre 4 et 10.

8. Composition de cire à épiler selon la revendication 7, **caractérisée en ce que** ledit tensioactif est du type non ionique.

9. Composition de cire à épiler selon l'une des revendications 7 et 8, **caractérisée en ce que** ledit tensioactif est un émulsionnant glucolipidique d'origine végétale.

10. Composition de cire à épiler selon l'une des revendications précédentes, **caractérisée en ce qu'**elle comprend : 72,5 % en poids de cire du type colophane, 26,5 % en poids de cire à base de sucre et 1 % en poids d'un émulsionnant du type alkylpolyglucoside.

11. Composition de cire à épiler selon l'une des revendications précédentes, **caractérisée en ce qu'**elle comprend en outre de 0 à 10 % en poids d'au moins un additif choisi dans le groupe comprenant les parfums, les huiles essentielles, les opacifiants, les antiseptiques, les analgésiques, les antibiotiques, les colorants, les plastifiants, les sels, seuls ou en mélange.
